Europäisches Patentamt

European Patent Office

Office européen des brevets

(11) Publication number: 0 344 770
A1

# EUROPEAN PATENT APPLICATION

(21) Application number: 89109929.3

(22) Date of filing: 01.06.89

(51) Int. Cl.⁴ A61B 5/05 , A61B 5/00

(30) Priority: 01.06.88 IL 86587

(43) Date of publication of application:
06.12.89 Bulletin 89/49

(84) Designated Contracting States:
AT BE CH DE ES FR GB GR IT LI LU NL SE

(71) Applicant: STATE OF ISRAEL-MINISTRY OF
AGRICULTURE
Agricultural Research Organization P.O. Box
6
Bet Dagan(IL)

Applicant: YEDA RESEARCH & DEVELOPMENT
COMPANY, LIMITED
P.O. Box 95
Rehovot 76100(IL)

(72) Inventor: Fischler, Henryk

deceased(IL)
Inventor: Lipkin, Ziv
4 Kipnes Street
Rehovot(IL)
Inventor: Aizinbud, Eliezer
35 Kaplinsky Street
Rishon Lezion(IL)
Inventor: Lehrer, Rami A.
17 Spinoza Street
Rehovot(IL)
Inventor: Tadmor, Amnon
4 Iris Street
Kiron(IL)
Inventor: Nachshon, Aharon
14 Harimon Street
Ramat Hasharon(IL)

(74) Representative: Kraus, Walter, Dr. et al
Patentanwälte Kraus, Weisert & Partner
Thomas-Wimmer-Ring 15
D-8000 München 22(DE)

(54) Device for telemetering living tissue impedance by radio means.

(57) Apparatus for measuring in vivo tissue imped-
ance comprising:
means (10) to be implanted into the body tissue
(12) for sensing the impedance thereof and for pro-
viding an output signal corresponding to the sensed
impedance to a remote location.
receiver means (62, 64, 66, 68) located at the
remote location for receiving the output signal and
means (80) associated with the receiver for
displaying the measured impedance value.

FIG.1
SH. 3 OF 3

## FIELD OF THE INVENTION

The present invention relates to radio telemetry apparatus and a method for monitoring physiological changes in animals.

## BACKGROUND OF THE INVENTION

It is known that animal tissues undergo physiological and pathological fluctuations in response to endogenous and environmental factors. These fluctuations result in measurable chances in the electrical properties of the tissues. It has been suggested, therefore, to monitor various electrical properties of the tissues for purposes of, inter alia, biomedical research, diagnosis of body conditions and animal husbandry.

In particular, it has been suggested to measure changes in electrical properties in the genitalia of dairy cows for detecting their ovulatory phase so as to avoid missing an oestrus cycle and thereby permit effective artificial insemination. U.K. Patent 1,402,677 discloses an ovulation monitor comprising electrode means for sensing the composition of vaginal fluids.

U.S. Patents 3,844,276 and 4,224,949 disclose sensing probes and methods for measuring the electrical resistance of vaginal mucus for oestrus detection in animals. These methods require repeated insertions of the sensing probe into the vaginal lumen at various time intervals. Among disadvantages in such methods are, however, that the animals have to be physically restrained each time the sensing probe is inserted in order to take a measurement.

In addition, repeated insertion of the sensing probe into the vaginal lumen is liable to result in irritation and inflammation of the vaginal mucosa. These methods also suffer from inaccuracies due to unstable contact between the probe and the vaginal mucosa.

It is known that among the measurable physiological changes in animal tissues is the state of hydration, a change in which results in a corresponding change in the electrical impedance of the tissues. Two articles entitled "The Electrical Conductivity inside the Bovine Vaginal Wall" (Anim. Prod. 1978, 26:61-65) and "Impedometric Properties of the Vulvar and Vaginal Tissues of Ewes during the Oestrus Cycle" (J. Reprod. Fert. 1981, 61:11-17) disclose that cyclic impedance fluctuations also exist inside the tissue of the vulvar and vaginal wall of cows and ewes, not only in the lumen. These fluctuations are measurable by use of implanted electrodes and hard-wire techniques.

For measurement of such changes in unrestrained animals, it has been suggested to use biotelemetry. E. J. Slater et al disclose, in an article entitled "A portable Radiotelemetry Receiver for Ambulatory Monitoring", (J. Biomed. Eng. July 1982, Vol. 4), a portable receiver including an aerial switching unit for use with "radio pills" for measuring pH inside the oesophagus using radiotelemetry techniques.

J.D.Pauley and M. Reite disclose, in an article entitled "A Microminiature Hybrid Multichannel Implantable Biotelemetry System", (Biotelemetry Patient Monitoring 8:163-172, 1981), a seven channel implantable PAM-FM biotelemetry transmitter and PAM demodulator that permit monitoring of body temperature, ECG, EOG, EMG and EEG.

The respective systems of E. J. Slater et al and of Pauley and Reite measure various physiological phenomena, but do not measure the state of hydration of animal tissues.

Disclosed in U.K. Patent 1,545,271 is telemetric apparatus for measuring biological parameters over a relatively long period of time comprising a radio transmitter located outside the body, a sensor capsule implanted inside the body and including circuitry for providing an output signal and a receiver located outside the body and responsive to signals provided by the sensor capsule.

German Offenlagungsschrift DE 29 41 363 A 1 discloses a device for the determination of the electrical conductivity of body fluids including an encapsulated radio probe sensor pinned in the lumen of the vagina of a cow and containing a storage unit for measured data, a telecommand receiver and a telemetric transmitter for transmitting an average value of data measured.

The respective telemetry devices disclosed in the above-mentioned U.K. and German patents are characterized by a number of disadvantages, among which is that as the probes for measuring oestrus are placed within the vaginal lumen, they must be removed before artificial insemination can be carried out. Furthermore, the prolonged attachment of the device to the sensitive vaginal mucosa causes irritation and inflammation thereof.

In the field of livestock identification, various methods exist, including branding and tagging, typically of the ears. Included among electronic identification apparatus for tagging animals are battery-powered transmitters in the form of ear tags, for example and passive systems such as are responsive to a remote trigger signal and such as those including magnetic tape.

A further system, disclosed by J. P. Hanson in 1974 and described in am article entitled "Electronic Identification of Livestock" (Proceedings of the International Federation of Automatic Control Symposium, Automatic Control for Agriculture and University of Saskatchewan, Saskatoon, Saskatchewan, Canada), includes a battery powered, encapsulated radio transmitter implanted orally with a balling gun into the rumen of an animal.

The capsule transmits a series of precoded electronic pulses from the animal (the pulse configuration being unique to each individual animal) which is received by a unit external to the animal, decoded and displayed. The transmitter is permanent and unalterable after implantation, has an interrogatable range up to 6 m and is noninjurious to the animal. The major disadvantage is that the transmitter is battery powered and has a limited useful life.

Yet a further system, described in a paper entitled "Development of a National Electronic Identification System for Livestock" by Dale M. Holm, (delivered at the session on "Electronic Aids in Dairy Research and Management", held July 30, 1979, during the 71st Annual Meeting of the ASAS, University of Arizona, Tucson), comprises a small electronic transponder that can be implanted under the skin of an animal. The transponder is generally intended to be implanted just beneath the skin on the back of the animal.

## SUMMARY OF THE INVENTION

It is an aim of the present invention to provide apparatus enabling continuous telemetric monitoring of a parameter reflecting fluctuations of body tissue hydration, namely, the electrical impedance thereof, the apparatus being more accurate in operation than prior art apparatus.

It is a further aid of the present invention to provide telemetric body tissue electrical impedance monitoring apparatus which includes a sensing portion which can remain in place in genital tissue of an animal for long periods of time, substantially without causing irritation or suffering to the animal.

There is provided, therefore, in accordance with an embodiment of the invention, apparatus for measuring in vivo tissue impedance comprising means to be implanted into body tissue for sensing impedance thereof and for providing to a remote location an output signal corresponding to the sensed impedance, receiver means located at the remote location for receiving the output signal and

means associated with said receiver means for displaying a sensible output of the sensed impedance.

Additionally in accordance with an embodiment of the invention, the apparatus for sensing impedance comprises apparatus for generating a low frequency exploratory signal, electrode apparatus coupled to the apparatus for generating for providing the exploratory signal to the tissue and apparatus for measuring an electrical potential difference across the tissue and for providing an output signal corresponding to the tissue impedance.

Further in accordance with an embodiment of the invention, the electrode apparatus comprises a bipolar electrode configured for electrical connection with the tissue.

Additionally in accordance with an embodiment of the invention, the apparatus for sensing and providing also comprises apparatus for storing in digital form a code corresponding to the identity of an animal of which the tissue forms a part and for providing to the receiver apparatus an output signal corresponding to the identity of the animal and apparatus for sensing the voltage supplied to the apparatus for sensing and providing and for providing to the receiver apparatus location an output signal corresponding to the sensed voltage.

Further in accordance with an embodiment of the invention, the receiver apparatus comprises apparatus for decoding the output signals and the apparatus for displaying a sensible output also comprises apparatus for displaying a sensible output of the code corresponding to the identity of the animal of which the tissue forms a part and of the supply voltage to the apparatus for sensing and providing.

Additionally in accordance with an embodiment of the invention, the apparatus for sensing impedance also comprises output signal modulation apparatus.

There is provided, in accordance with an alternative embodiment of the invention, a method of measuring in vivo impedance of animal tissue comprising the steps of implanting within the tissue an electrode assembly having two electrodes; applying to the tissue via the electrode assembly a low frequency exploratory signal; measuring an electrical potential difference through the tissue across the two electrodes; providing to a remote location, by apparatus associated with the electrode assembly, an output signal corresponding to the measured electrical potential dfference; receiving and decoding at the remote location the output signal; and providing at the remote location sensible output indications corresponding to a value of tissue impedance corresponding to the electrical potential difference measured therethrough.

Additionally in accordance with the alternative embodiment of the invention, the method also includes the additional steps of accessing a serial number stored by memory apparatus and corresponding to the identity of an animal of which the tissue forms a part; providing to the remote location an output signal corresponding to the serial number; supplying from an independent source a voltage to the electrode assembly; monitoring the supply voltage to the electrode assembly; providing to the remote location an output signal corresponding to the supply voltage; receiving and decoding at the remote location the output signals corresponding, respectively, to the serial number and the supply voltage; and providing at the remote location sensible output indications corresponding, respectively, to the output signals corresponding to the serial number and the supply voltage.

## BRIEF DESCRIPTION OF THE DRAWINGS

The present invention will be understood and appreciated more fully from the following detailed description, taken in conjunction with the drawings, in which:

Fig. 1 is a block diagram illustration of impedance sensor and transmitter apparatus, constructed and operative in accordance with a preferred embodiment of the invention; and

Fig. 2 is a block diagram illustration of receiver apparatus and trigger apparatus, useful in conjunction with the apparatus of ·Fig. 1.

## DETAILED DESCRIPTION OF A PREFERRED EMBODIMENT

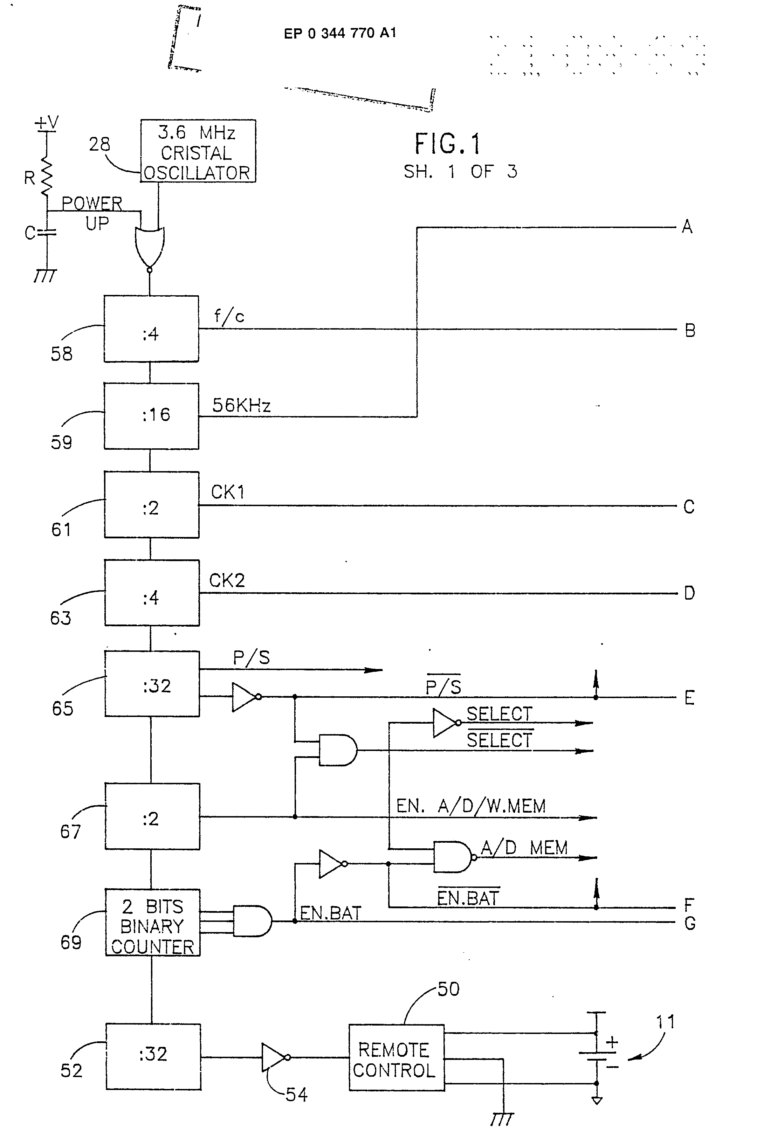

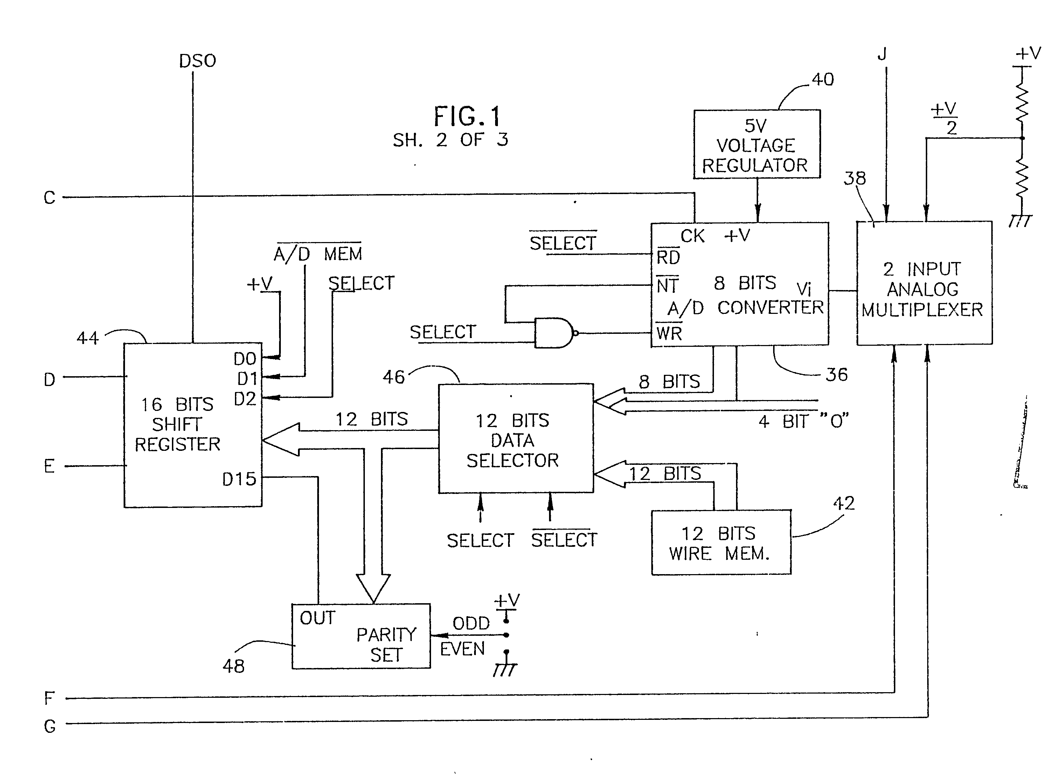

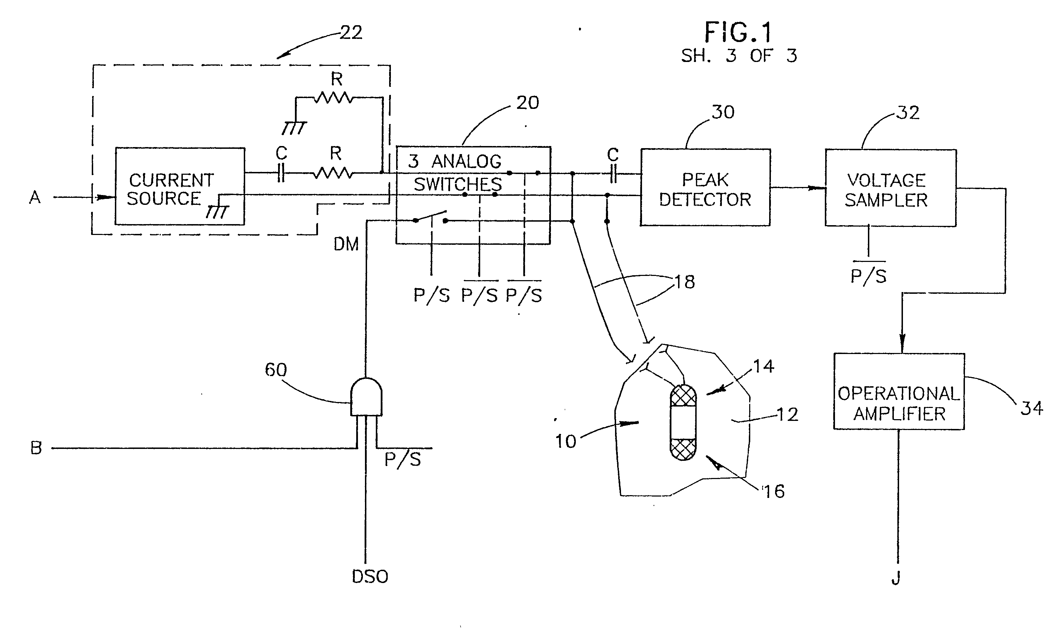

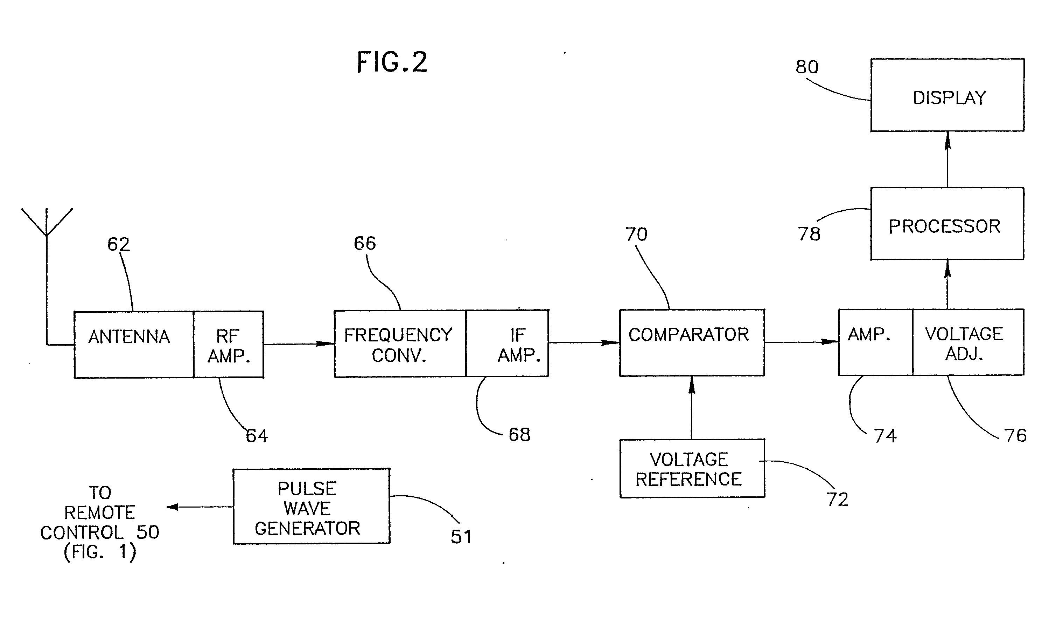

Referring to Figs. 1 and 2, there is shown a radiotelemetry system for providing on-line in vivo measurements of the absolute vulvar or vaginal tissue impedance of animals. The system is also operative to identify an animal by a serial number, to measure a supply voltage to the sensing apparatus and to transmit the information to a remote monitoring station.

Referring particularly to Fig. 1, there is provided sensor and transmitter apparatus comprising an electrode assembly referenced generally 10, configured for direct coupling to a portion of genital tissue 12 to be monitored. Typically, the electrode assembly is implanted into the vulvar tissue or into the vaginal wall. Assembly 10 is preferably coupled to tissue 12 by implantation therein and is powered by means of an independent source, such as a battery, shown by reference numeral 11. It will be appreciated by those skilled in the art that electrode assembly 10 is configured so as to contain all the apparatus schematically shown and described in conjunction with Fig. 1.

According to a preferred embodiment, electrode assembly 10 is bipolar and comprises a pair of electrodes 14 and 16 in the form of hemispheres separated from each other by a medical grade epoxy resin which is effective to bind together the electrodes in the form of a capsule. Electrodes 14 and 16 are coupled, by means of connecting leads 18, to analog switches 20 which are connected to a current source, referenced generally 22. The respective surfaces of electrodes 14 and 16 are preferably roughened so as to provide a good and stable electrical connection between electrode assembly 10 and tissue 12.

According to an alternative embodiment of the invention, electrode assembly 10 is tetra-polar rather than bipolar.

Electrodes 14 and 16 may be made of any suitable tissue-compatible material, such as medical-grade stainless steel or titanium. A suitable electrode capsule comprises a pair of hemispherical electrodes, each having a radius of 4 mm and, therefore, a surface area of substantially 1 square centimeter. The electrodes are separated from each other by a distance of 20 mm by a volume of medical grade Epoxy Resin such as Stycast 1267 of Emerson & Cuming Europe N.V..

The epoxy resin isolates connecting leads 18 and prevents their direct contact with tissue 12 with which they are incompatible and also prevents shorting of the leads by contact with body fluids. Outside the capsule, leads 18 are enclosed by medical grade tubing (not shown) which typically is Silastic tubing manufactured by Dow Corning.

There is provided an oscillator 28, which typically is a crystal oscillator, providing electrical signals, typically at a frequency of 3.6 MHz. The pulse is divided by frequency dividers shown by respective reference numerals 58, 59, 61, 63, 65, 67, 69 and 52.

Frequency divider 59 is operative to generate an exploratory low frequency signal which is converted to a constant current signal by means of current source 22. The constant current has a density of preferably between 10 microamps per square centimeter and 1 milliamp per square centimeter, with preferably a peak value provided to electrodes 14 and 16 of 0.5 milliamp per square centimeter during impedance measurement.

It is a particular feature of the present invention that the exploratory low frequency signal, being in the range of 10 KHz to 1 MHz and preferably of 56 KHz, is utilized for impedance measurement.

In operation, the exploratory low frequency signal is applied to tissue 12 via electrode assembly 10. The potential drop of the signal through the tissue between electrodes 14 and 16 is proportional to the absolute tissue impedance at the time of measurement. It has been found that at the signal frequency used, polarization impedance of electrodes 14 and 16 is negligible when compared with the measured impedance of the tissue. This has been determined in experiments carried out on electrodes 14 and 16 embedded in physiological phosphate buffered saline.

In addition, it has been found that at a frequency of 56 KHz, measuring complications and inaccuracies resulting from parasitic capacitances within the measuring system are substantially avoided.

It will be appreciated that the peak value of 0.5 milliamp per square centimeter provided to electrodes 14 and 16 during impedance measurement provides linear measuring conditions. This peak value lies within the range of values giving linear behaviour of tissues as known in the art and it is below the threshold of nonlinear behavior of tissues, which has been found to be about 1 milliamp per square centimeter. It has further been found that at the level of current density used, an animal whose tissue is being monitored does not experience any unpleasant sensations such as contraction or twitching of the tissue, nor is the tissue itself harmed.

As will be appreciated, it is preferable that the density of signals used in measuring tissue impedance are maintained within the range giving rise to linear tissue behavior, as in this range the tissue impedance measured is not dependent upon the density of the exploratory signal. This is in contrast to measurements wherein the density of the signals used are in the range of nonlinear tissue behavior, wherein the value of the measured impedance is in inverse proportion to the value of the signal density.

When the peak current, as described above, is provided to electrodes 14 and 16, there is established therebetween an electrical potential difference directly proportional to the impedance of the tissue. It has been found that at the peak current value, there is established through tissue 12 a potential difference lying in the range 25 to 75 millivolts, which corresponds to a tissue impedance range of 100 to 300 Ohms. The potential difference across the electrodes is provided to peak detector means 30 which is operative to determine the peak value of the potential difference.

A voltage sampler 32 is operative to store the maximum voltage value output from peak detector 30 and this voltage is amplified to a level of several volts by an operational amplifier 34. Amplifier 34 may be any suitable amplifier that complies with stability requirements for the current provided to electrode 10. Preferably, amplifier 34 is stable to the extent that the total gain thereof varies by no more than + - 1% for a + -10% variation of the supply voltage within a temperature range of 25 to 40 degrees celsius.

The output voltage from amplifier 34, which is proportional to the tissue impedance value, is provided, via an analog multiplexer 38 to an 8 bit analog to digital converter 36. Converter 36 is operative to alternately receive via multiplexer 38 two levels of voltage, one of which is the measured voltage proportional to the tissue impedance and the other of which corresponds to the voltage of battery 11. Converter 36 is powered by a 5 V voltage regulator 40 so as to ensure that it is not influenced by changes in the voltage supplied from battery 11.

A 12 bit data selector 46 is operative to receive, in alternating fashion, respective inputs provided from converter 36 and from a 12 bit wire memory 42 containing the electrode capsule number and, therefore, a serial number of the animal being monitored. Output from selector 46 is provided to a 16 bit shift register 44. According to the present embodiment, memory 42 may be programed to store any of 4096 different capsule serial numbers by which the particular animal being monitored may be identified. The capsule number is transmitted to the receiving apparatus (Fig. 2) alternately with a value corresponding to the tissue impedance.

It is appreciated that interference induced by, for example, atmospheric disturbances, may affect the information received by the receiving apparatus. There is thus transmitted with the impedance data, the supply voltage value and the capsule serial number, a digital code which is related to the transmitted information and thus indicates whether or not it has been subject to interference. The digital code is a one bit digital code provided to register 44 by a parity set 48.

Alternatively, or in addition to the digital code, there may also be transmitted respective codes transmitted with the three types of information and indicating the type of information being transmitted, namely, whether it is an indication of tissue impedance, the animal identity number or the supply voltage. The code is typically carried by bits D0, D1 and D2 of shift register 44.

According to a preferred embodiment, not more than about 1 microamp is continuously supplied to the system shown in Fig. 1. An external short trigger of between 5 milliseconds and 1 second at a frequency of 2. 4 MHz is provided by externally operated pulse wave generator apparatus, referenced 51, to a coil within a remote control unit 50 which causes the system to operate for about 1.17 seconds, after which it is shut down automatically by divider 52 and an inverter 54. An example of suitable pulse wave generator apparatus 51 is Function Generator 33121, manufactured by Hewlett Packard.

According to the shown embodiment, the signals to be transmitted to the receiving apparatus (Fig. 2) undergo pulse modulation by an AND gate 60. It is known that electromagnetic waves are absorbed by body tissues to a degree that depends on the frequency of the waves. The carrier signal frequency may range between about 10 KHz and 100 MHz, although a preferred frequency is 900 KHz. The carrier signal is provided via frequency divider 58. To provide frequency stability of the carrier signal, oscillator 28 is preferably a crystal oscillator having a stability of 0.0001% with time.

Data received by register 44 is provided in serial fashion to AND gate 60 and transmitted to the environment via electrode assembly 10 which serves additionally as an antenna. According to an alternative embodiment of the invention, the data may be transmitted by magnetic induction through the coil of remote control 50.

In summary, the above-described system is operative in two modes, namely, a first mode in which information is gathered, the information including the tissue impedance of an animal being monitored, checking the battery voltage and collecting data from wire memory 42 and a second mode in which the gathered information is transmitted to the receiving apparatus shown in Fig. 2.

As described above, the transmission includes three categories of information which are transmitted several times and, preferably, in the following order:

    a. the serial number of the capsule, corresponding to the animal being monitored,

    b. the impedance of the tissue and

    c. the supply voltage, which indicates if the system is working properly.

In accordance with the present invention, any one of a variety of power supplies may be employed as an alternative to battery 11. The type of power supply used may depend upon the location of the sensor apparatus with respect to the animal and upon the period of time over which monitoring is sought to be carried out. According to a preferred embodiment, batteries are used, typical batteries being lithium, mercury or alkaline.

According to an alternative embodiment, during operative periods and with the addition to the sensor apparatus of suitable passive conversion apparatus, power may be provided externally, such as by electromagnetic induction. This type of external power supply may be provided particularly when space within the capsule is scarce, such as when it is to be implanted in a sheep or when it is wished to carry out monitoring over a relatively long period.

Referring now to Fig. 2, there is shown, in block diagram form, receiver apparatus useful in conjunction with the transmitter apparatus of Fig. 1. The receiver apparatus comprises an antenna 62, which is preferably a ferrite antenna. The maximum distance that the antenna may be placed from the transmitter so as to ensure satisfactory reception depends on the radiation power of the carrier signal, the frequency at which it is transmitted and whether the sensor apparatus is implanted beneath the surface of the skin of the animal and, if so, at what depth. A distance of one meter has been found to be satisfactory although a different distance may also be suitable.

Signals received by antenna 62 are amplified by a conventional RF amplifier 64 and are converted by a frequency converter 66 into standard intermediate frequency signals of typically 455 KHz, which carry all the pulse modulated information of the transmitted signals. The intermediate frequency signals are filtered out by an intermediate frequency amplifier 68.

The filtered-out signals are converted to pure digital signals by means of a comparator 70, which compares the signals to a known voltage reference provided by apparatus 72. The pure digital signals are then amplified by an amplifier 74 and, by means of a voltage adjuster 76, the respective voltage levels of the signals are then matched to the voltage level of a processor 78 to which the signals are provided.

The signals received by processor 78 are demodulated thereby and are decoded to determine the contents of each signal, namely, if it contains the capsule serial number, the tissue impedance or the battery voltage. The processor further determines whether or not the information has been altered during transmission by external influences. The data thus decoded may be stored in the processor and may be retrieved and displayed by a monitor 80 or by any other sensible display means.

While, according to the specific embodiment described above, the apparatus of the invention has been described as being useful for identifying an animal and determining the oestrus thereof for the purpose of artificial insemination, the apparatus may alternatively be used for measurement of any other body state having a measurable effect on tissue impedance. Among these states are hormonal changes, that may indicate the imminence of calving and various infections or illnesses known to affect the tissue impedance.

It will be appreciated by persons skilled in the art that the present invention is not limited to what has been shown and described hereinabove. The scope of the invention is limited, rather, solely by the claims, which follow:

## Claims

1. Apparatus for measuring in vivo tissue impedance comprising:
means to be implanted into body tissue for sensing impedance thereof and for providing to a remote location an output signal corresponding to the sensed impedance,
receiver means located at the remote location for receiving the output signal and
means associated with said receiver means for displaying a sensible output of the sensed impedance.

2. Apparatus according to claim 1, and wherein said means for sensing impedance comprises:
means for generating a low frequency exploratory signal,
electrode means coupled to said means for generating for providing the exploratory signal to the tissue and
means for measuring an electrical potential difference through the tissue and for providing an output signal corresponding to the tissue impedance.

3. Apparatus according to either of claims 1 or 2, and wherein said means for sensing impedance is encapsulated in a tissue compatible material.

4. Apparatus according to either of claims 2 or 3, and wherein the exploratory signal has a frequency of between 10 KHz and 1 MHz.

5. Apparatus according to either of claims 2 or 3, and wherein the exploratory signal has a frequency of 56 KHz.

6. Apparatus according to either of claims 2 or 3, and wherein said means for generating is operative to generate signals having a frequency of 56 KHz at a substantially constant current density of between 10 microamps per square centimeter and 1 milliamp per square centimeter.

7. Apparatus according to either of claims 2 or 3, and wherein said means for generating is operative to generate signals having a frequency of 56 KHz at a substantially constant current density of 500 microamps per square centimeter.

8. Apparatus according to any of claims 2 to 7, and wherein said electrode means comprises a bipolar electrode assembly configured for electrical connection with the tissue.

9. Apparatus according to any of claims 2 to 7, and wherein said electrode means comprises a tetra-polar electrode assembly configured for electrical connection with the tissue.

10. Apparatus according to any of the preceding claims, and wherein said means for sensing impedance and for providing an output signal also comprises means for storing in digital form a code corresponding to the identity of an animal of which the tissue forms a part and for providing to said receiver means an output signal corresponding to the identity of the animal.

11. Apparatus according to any of the preceding claims, and wherein said means for sensing impedance and for providing an output signal further comprises:
voltage supply means and
means for sensing the voltage supplied to said means for sensing impedance and for providing to said receiver means an output signal corresponding to the sensed voltage.

12. Apparatus according to any of the preceding claims, and wherein said means for sensing impedance also comprises means for modulating the output signals.

13. Apparatus according to claim 12, and wherein said means for modulating is operative to modulate the output signals to a frequency of between 10 KHz and 100 MHz.

14. Apparatus according to claim 13, and wherein said means for modulating is operative to modulate the output signals to a frequency of 900 KHz.

15. Apparatus according to any of claims 10 to 14, and wherein said receiver means comprises means for decoding the output signals and said means for displaying a sensible output also comprises means for displaying a sensible output of said code corresponding to the identity of the animal of which the tissue forms a part.

16. Apparatus according to any of claims 10 to 15, and wherein said receiver means comprises means for decoding the output signals and said means for displaying a sensible output further comprises means for displaying a sensible output of the supply voltage to said means for sensing and providing.

17. Apparatus according to either of claims 15 or 16, and wherein said means for displaying comprises digital display means.

18. Apparatus according to any of the preceding claims, and wherein the body tissue into which said means for sensing impedance is to be implanted is genital tissue of an animal.

19. A method of measuring in vivo impedance of animal tissue comprising the steps of:
implanting within the tissue apparatus for sensing the impedance thereof and including at least two electrodes;
applying to the tissue via said at least two electrodes a low frequency exploratory signal;
measuring an electrical potential difference through the tissue and across said at least two electrodes;

providing to a remote location, by means associated with said electrode assembly, an output signal corresponding to the measured electrical potential difference;

receiving and decoding at the remote location the output signal; and

providing at the remote location sensible output indications corresponding to a value of tissue impedance corresponding to the electrical potential difference measured therethrough.

20. A method according to claim 19, and wherein said at least two electrodes comprise three electrodes.

21. A method according to either of claims 19 or 20, and wherein the step of applying a low frequency exploratory signal comprises the step of applying an exploratory signal having a frequency of between 10 KHz and 1 MHz.

22. A method according to claim 21, and wherein the exploratory signal has a frequency of 56 KHz.

23. A method according to claim 22, and wherein the step of applying an exploratory signal at 56 KHz comprises the additional step of generating the signal at a substantially constant current density of between 10 microamps per square centimeter and 1 milliamp per square centimeter.

24. A method according to claim 23, and wherein the signal is generated at a substantially constant current density of 500 microamps per square centimeter.

25. A method according to any of claims 19 to 24, and also including the additional steps of:

accessing a serial number stored by memory means and corresponding to the identity of an animal of which the tissue forms a part;

providing to the remote location an output signal corresponding to said serial number;

receiving and decoding at the remote location said output signal corresponding to said serial number; and

providing at the remote location sensible output an indication corresponding to said serial number.

26. A method according to any of claims 19 to 25, and further including the additional steps of:

supplying from an independent source a voltage to said apparatus for sensing tissue impedance;

monitoring the supply voltage to said impedance sensing apparatus;

providing to the remote location an output signal corresponding to the supply voltage;

receiving and decoding at the remote location said output signal corresponding to the supply voltage; and

providing at the remote location a sensible output indication corresponding to the supply voltage.

27. A method according to any of claims 19 to 26, and also including the step of modulating the output signals.

28. A method according to claim 27, and wherein said step of modulating comprises modulating the output signals to a frequency of between 10 KHz and 100 MHz.

29. A method according to claim 28, and wherein said step of modulating comprises modulating the output signals to a frequency of substantially 900 KHz.

30. A method according to any of claims 19 to 29, and wherein said step of implanting comprises the step of implanting said impedance sensing apparatus into genital tissue of an animal.

FIG.1
SH. 1 OF 3

FIG.1
SH. 2 OF 3

FIG.1
SH. 3 OF 3

EP 0 344 770 A1

# FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,X | DE-A-2 941 363 (D.F.V.L.R.)<br>* Pages 1-2; claims 1,4,6; page 5, lines 1-17; page 6, lines 1-31; page 9, line 18 - page 10, line 14; figures 1-7 * | 1,3,8, 15-19, 27,30 | A 61 B 5/05<br>A 61 B 5/00 |
| D,A | US-A-4 224 949 (N.R. SCOTT et al.)<br>* Abstract; column 1, lines 48-68; column 3, lines 20-24,57-64; column 4, lines 37-40; column 7, lines 19-31; column 10, lines 53-63; figures 1-4,20,24 * | 1-4,8,9 ,11,18, 19,21, 30 | |
| X,P | US-A-4 784 155 (P.A. MILLS)<br>* Abstract; column 2, lines 38-64; column 3, lines 8-39; column 4, lines 50-60; column 5, lines 29-58; figures 1-3 * | 1-3,8, 12,18, 19,27, 30 | |
| A | US-A-4 399 821 (D.L. BOWERS)<br>* Abstract; column 1, lines 42-65; column 2, line 64 - column 3, line 28; column 4, line 59 - column 6, line 41; figure * | 1-3,10, 12,15, 19,25 | TECHNICAL FIELDS SEARCHED (Int. Cl.4)<br><br>A 61 B |
| A | US-A-4 686 998 (A. ROBBINS)<br>* Abstract; column 3, lines 16-39; column 7, line 62 - column 8, line 9; column 9, lines 52-57; column 12, lines 40-62; column 13, lines 38-49; column 15, lines 25-33; figures 1-7 * | 1,2,4,5 ,11,16, 17,19, 21,22, 26,27 | |

The present search report has been drawn up for all claims

| Place of search | Date of completion of the search | Examiner |
|---|---|---|
| THE HAGUE | 08-09-1989 | RIEB K.D. |